# EUROPÄISCHE PATENTSCHRIFT

(11) **EP 0 427 908 B1**
(45) Veröffentlichungstag und Bekanntmachung des Hinweises auf die Patenterteilung: **07.09.1994**
(21) Anmeldenummer: 90109047.2
(22) Anmeldetag: 14.05.1990
(51) Int. Cl.: G06M 11/00, G06M 1/10

(54) **Partikelzähler**
Particle counter
Compteur de particules

(30) Priorität: 24.10.1989 DE 8912584 U
(43) Veröffentlichungstag der Anmeldung: 22.05.1991
(73) Patentinhaber: HYDAC TECHNOLOGY GMBH, D-66280 Sulzbach (DE)
(72) Erfinder: Hornung, Dieter, Dr.,Dipl-Phys, D-6797 Waldmohr (DE); Sahner, Paul, Dipl-Ing., D-6638 Dillingen/Saar (DE); Korb, Jürgen, Dipl.-Ing., D-6682 Ottweiler-Fürth (DE); Huppert, Michael, Dipl.-Ing., D-6601 Heusweiler (DE); Funk, Norbert, Dipl.-Ing., D-6670 St. Ingbert (DE)
(74) Vertreter: Patentanwälte Bartels und Partner

(56) Entgegenhaltungen:
- GB-A- 1 389 247
- US-A- 3 924 951
- US-A- 4 157 468
- Prospekt "Pacific Scientific", Instrument Division, HRC 86082, 5 Seiten, eingegangen am 30.10.93 mit Brief vom 29.10.93.

## Beschreibung

Die Erfindung betrifft einen nach dem Abdunklungsverfahren arbeitenden Partikelzähler, insbesondere zum Zählen von opaken Partikeln in einem Fluidstrom eines Hydraulik- oder Schmiersystems, mit einer Lichtschranke, deren Lichtstrahl einen Meßkanal für das Fluid quer zur Kanallängsrichtung durchdringt, und einer dem Empfänger der Lichtschranke nachgeschalteten Auswerteelektronik.

Die bekannten Partikelzähler dieser Art sind aufwendige Laborgeräte hoher Präzision. Sie besitzen eine hochwertige Präzisionsoptik, was mit dazu beiträgt, daß diese Geräte sehr empfindlich gegen äußere Einflüsse, insbesondere gegen Erschütterungen, Luftfeuchte und Temperaturschwankungen, sind. Der hohe Preis ist aber auch durch eine aufwendige Bedien-und Auswerteelektronik bedingt. Für den Industrieeinsatz, beispielsweise in einem Hydrauliksystem, kommen deshalb diese Partikelzähler nicht in Frage.

Der Erfindung liegt die Aufgabe zugrunde, einen Partikelzähler der eingangs genannten Art zu schaffen, der besonders für den rauhen Industrieeinsatz geeignet ist. Diese Aufgabe löst ein Partikelzähler mit den Merkmalen des Anspruches 1. Ein solcher Partikelzähler ist dank der einfachen, in den Sensorkörper eingebetteten Elektronik erschütterungsfest und in einem großen Tempersturbereich funktionsbereit. Durch die Anordnung der Auswerteelektronik in einem Schutzgehäuse, das vorzugsweise ein solches der Schutzart IP 65 ist, läßt sich die erforderliche Störunanfälligkeit der Elekronik mit geringem Aufwand erreichen. Da der erfindungsgemäße Partikelzähler alle für seine Funktion erforderlichen Komponenten in einer Baueinheit vereinigt, ist er sehr bedienungsfreundlich, das heißt, zu einer Installation und Inbetriebnahme ist kein geschultes Fachpersonal erforderlich. Ferner führt der erfindungsgemäße Zähler zu einem kompakten Geräteaufbau. Schließlich erfüllt der erfindungsgemäße Zähler dank des einfachen Aufbaus sowohl seiner Optik als auch seiner Elektronik die Forderungen nach geringen Kosten.

Durch die Verwendung des Abdunklungsverfahrens ist es möglich, auch die Partikelgröße zu bestimmen, weil die Abdunklung um so stärker ist, je größer die Partikel sind. Es lassen sich deshalb auf elektronischem Wege in einfacher Weise Partikelklassen definieren. Bei einer bevorzugten Ausführungsform ist deshalb die Ermittlung von wenigen, beispielsweise zwei bis fünf, fest eingestellten, kumulativen Partikelklassen möglich.

Vorteilhafterweise ist der Meßkanal gemäß Anspruch 2 ausgebildet, da hierdurch Reflexionen an den Wänden des Strömungskanales praktisch vermieden werden, woduch die Sensordetektivität sich erheblich verbessern läßt.

Aus Gründen einer guten Sensordetektivität weist ferner die Beleuchtungsfaser die Merkmale des Anspruches 3 auf. Demselben Zwecke dient ein Ausbildung der Empfangsfaser gemäß Anspruch 5. Eine gute Schärfentiefe erhält man durch die Merkmale des Anspruches 4.

Als Lichtquelle, deren Licht von der Beleuchtungsfaser zum Meßkanal geleitet wird, ist bei einer bevorzugten Ausführungsform eine Leuchtdiode vorgesehen, die ein äußerst intensives Infrarotlicht abstrahlt.

Dem Empfänger der Lichtschranke, bei dem es sich vorzugsweise um eine Photodiode handelt, ist bei einer bevorzugten Ausführungsform ein Signalvorverstärker nachgeschaltet, dem wiederum zweckmäßigerweise eine Signalvorverarbeitungsstufe nachgeschaltet ist.

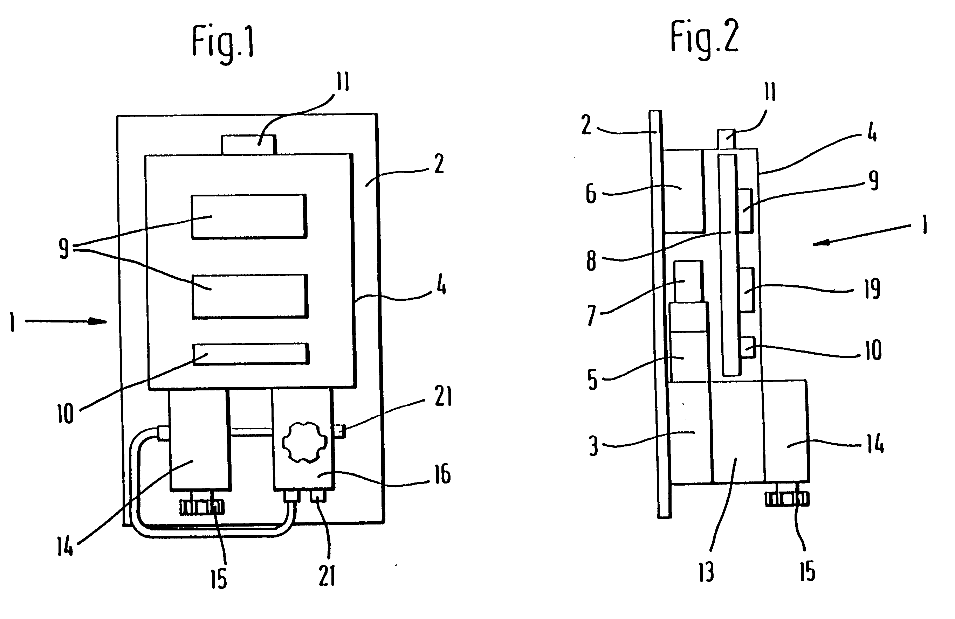

Um bequem die Partikelzahl ausgeben und den Funktionszustand des Zählers überwachen zu können, ist der Zähler bei einer bevorzugten Ausführungsform gemäß Fig. 1 ausgebildet. Im Anzeigefenster erscheint hier das Zählergebnis. Das Diagnosefenster enthält Anzeigelemente, insbesondere Leuchtdioden, welche anzeigen, ob der Zähler ordnungsgemäß arbeitet oder ob eine Überprüfung einer oder mehrerer Funktionen notwendig ist.

Um die Handhabung und insbesondere auch die Montage am Einsatzort zu erleichtern, sind vorteilhafterweise der Sensor und das Gehäuse auf einer gemeinsamen Grundplatte angeordnet. Für den Einsatz braucht deshalb nur noch der Fluidanschluß und, sofern die Elektronik nicht von einer Batterie, sondern von einem Netzteil gespeist wird, der elektrische Anschluß hergestellt zu werden.

Zusätzlich zu dem Sensor kann ein Volumenstromregler vorgesehen sein, mittels dessen eine genaue Einstellung des den Meßkanal durchströmenden Volumens möglich ist. In einem solchen Falle ist es vorteilhaft, für den Volumenstromregler ein Vorfilter vorzusehen.

Sofern der Zähler an eine Druckleitung angeschlossen werden muß, ist es zweckmäßig, eine Druckabsenkungseinheit vorzusehen, die vorteilhafterweise gemäß Anspruch 15 ausgebildet ist.

Im folgenden ist die Erfindung anhand eines in der Zeichnung dargestellten Ausführungsbeispieles im einzelnen erläutert.

### Es zeigen

- Fig. 1: eine Draufsicht auf das Ausführungsbeispiel,
- Fig. 2: eine Seitenansicht des Ausführungsbeispiels,
- Fig. 3: einen vergrößert dargestellten Schnitt durch den Meßkanal und die Optik der Lichtschranke.

Ein als Ganzes mit 1 bezeichneter "Low-Cost-Partikelzähler" für rauhen Industrieeinsatz, insbesondere in Hydraulik- oder Schmiersystemen, weist eine im Ausführungsbeispiel rechteckförmige Grundplatte 2 auf. Auf dieser Grundplatte 2 ist ein quaderförmiger Sensor 3 angeordnet, welcher an ein Gehäuse 4 der Schutzart IP 65 anschließt, das lösbar mit der Grundplatte verbunden ist. Das an den Sensor 3 anschließende Gehäuse 4 enthält eine Signalvorverstärkungsstufe 5, ein Netzteil 6, das die Stromversorgung für die Elektronik und den Sensor 3 liefert, sowie eine Signalvorverarbeitungsstufe 7 und einen Auswerte-und Anzeigemodul 8.

In seiner parallel zur Grundplatte 2 liegenden Oberseite weist das Gehäuse 4 drei rechteckförmige Durchbrüche auf, von denen zwei als Anzeigefenster 9 und einer als Diagnosefenster 10 dienen. In den beiden Anzeigefenstern 9 werden die Meßergebnisse angezeigt. Das Diagnosefenster 10 gibt den Blick frei auf nicht dargestellte Leuchtdioden, welche eine Funktionsüberwachung des Zählers ermöglichen. Beispielsweise zeigen diese Leuchtdioden an, ob der Zähler betriebsbereit oder nicht betriebsbereit ist, ob der Diodenstrom der Leuchtdiode des Sensors 3 zu klein oder zu groß ist, ob eine Übersteuerung vorhanden ist und ob in Bälde eine Wartung, insbesondere eine Reinigung des Sensors 3, erforderlich ist.

Im Ausführungsbeispiel ist ferner das Gehäuse 4 noch mit einer Schnittstelle 11 versehen, um die Meßdaten zu einer Erfassungsstelle weiterleiten und mit einer Automatisierungseinheit oder dergleichen kommunizieren zu können.

Mit den vorstehend beschriebenen Baugruppen ist der Zähler voll funktionsfähig. Im Ausführungsbeispiel ist jedoch der Ausgang eines Meßkanales 12 des Sensors 3 mit einem Vorfilter 13 verbunden, dem ein Volumenstromregler 14 nachgeschaltet ist. Das Vorfilter 13 und der Volumenstromregler 14 haben im Ausführungsbeispiel die gleiche Blockform wie der Sensor 3 und sind über diesem gestapelt angeordnet, wie Fig. 2 zeigt. Ein Handrad 15 dient der Einstellung des Volumenstromreglers 14, mittels dessen der durch den Meßkanal 12 fließende Volumenstrom genau eingestellt werden kann.

Weiterhin ist im Ausführungsbeispiel auf der Grundplatte 2 neben dem aus dem Sensor 3, dem Vorfilter 13 und dem Volumenstromregler 14 gebildeten Stapel eine Druckabsenkungseinheit 16 angeordnet, damit der Zähler an eine Druckleitung angeschlossen werden kann. Die Druckabsenkungseinheit enthält ein Drosselventil und ein Druckventil. Wie Fig. 1 zeigt, sind der Eingang des Sensors 3 und der Ausgang des Volumenstromreglers 14 über je eine Leitung mit der Druckabsenkungseinheit 16 verbunden, welche zwei Anschlüsse 21 aufweist, über welche der Partikelzähler 1 mit dem Hydraulik- oder Schmiersystem verbunden wird.

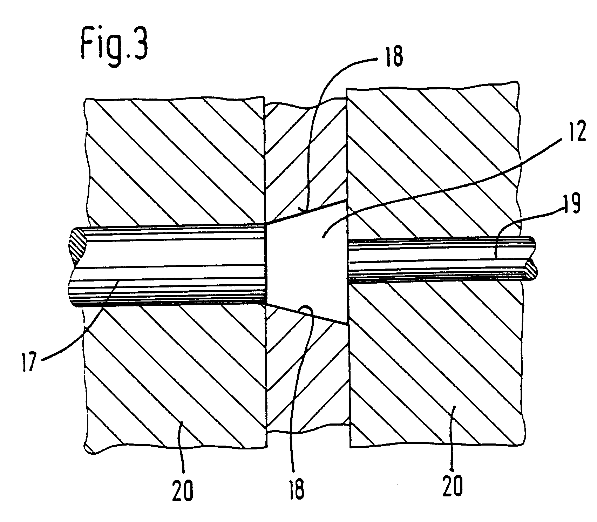

Der Meßkanal 12 im Sensor 3 verengt sich gegen die Meßstelle hin und hat zumindest dort die Querschnittsform eines Trapezes, wie dies Fig. 3 zeigt. Die kürzere der beiden zueinander parallelen Seiten wird von der Endfläche einer durch eine Glasfaser gebildeten Beleuchtungsfaser 17 gebildet, die im Ausführungsbeispiel einen Außendurchmesser von 300 µ hat. Das andere Ende der Beleuchtungsfaser 17 empfängt ein äußerst intensives Infrarot-Licht von einer als Lichtquelle dienenden Leuchtdiode. Die die beiden Trapezseiten bildenden, ebenen Flächen 18 des Strömungskanales 12 schließen beide mit der Längsachse des an den Meßkanal 12 angrenzenden, geradlinigen Endabschnittes der Beleuchtungsfaser 17 einen Winkel ϑ_{A} ein, der gleich dem Aperturwinkel der Beleuchtungsfaser gewählt ist. Dadurch wird verhindert, daß das aus der Endfläche der Beleuchtungsfaser 17 austretende Licht an den ebenen Flächen 18 reflektiert wird. Die Vermeidung solcher Reflexionen erhöht die Detektivität des Partikelzählers 1 erheblich.

Die zur Endfläche der Beleuchtungsfaser 17 parallel verlaufende Begrenzungsfläche des Kanals 12 wird teilweise durch die Endfläche einer Empfängerfaser 19 gebildet, deren geradliniger, an den Meßkanal 12 angrenzender Endabschnitt gleichachsig zur Beleuchtungsfaser 17 angeordnet ist. Der Durchmesser der durch eine spezielle Glasfaser gebildeten Empfängerfaser 19 ist etwa halb so groß wie der Durchmesser der Beleuchtungsfaser 17. Das andere Ende der Empfängerfaser 19 ist an eine Photodiode angekoppelt, welche den Empfänger der Miniaturlichtschranke bildet, deren Lichtstrahl den Meßkanal 12 quer zu dessen Längsrichtung durchdringt.

Die Beleuchtungsfaser 17 und die Empfängerfaser 19 sind vollständig mit Hilfe von Epoxidharz in den Sensorkörper 20 eingebettet. Dieser Sensorkörper 20 enthält auch den Meßkanal 12 sowie die als Lichtquelle dienende Leuchtdiode und die als Empfänger dienende Photodiode. Der Sensor 13 ist deshalb gegen Feuchtigkeit und andere äußere Einflüsse geschützt und außerdem unempfindlich gegen Erschütterungen und dergleichen. Der Sensorkörper 20 befindet sich in einem Schutzgehäuse.

Die Arbeitsweise des Partikelzählers ist folgende:

Der Meßkanal 12 wird ständig von dem Fluidstrom, welcher die zu zählenden Partikel mit sich führt, laminar durchströmt. Der aus der Beleuchtungsfaser 17 austretende und den Meßkanal 12 durchdringende, dünne Lichtstrahl der Lichtschranke ist auf die Empfängerfaser 19 gerichtet. Dank des sehr geringen Querschnittes des Meßkanales 12 in Höhe der Lichtschranke kann man davon ausgehen, daß die meisten im Fluid mitgeführten Partikel vom Lichtstrahl erfaßt werden und die Empfangsfläche der Empfängerfaser 19 in Abhängigkeit von der Partikelgröße mehr oder weniger stark abschatten. Das in die Empfängerfaser 19 eintretende Licht gelangt zu der Photodiode, in welcher die Helligkeitsschwankungen an der Empfangsfläche der Empfängerfaser 19 in sich entsprechend ändernde elektrische Signale umgewandelt werden. Zwischen zwei aufeinanderfolgenden Partikeln wird das auf die Empfangsfläche der Empfängerfaser 19 treffende Licht nur durch die Trübung des Fluids abgeschwächt. Die Signalvorverstärkungsstufe 7 ermöglicht es, unterschiedlich stark getrübte Flüssigkeiten zu untersuchen, da sie das Ausgangssignal der Photodiode automatisch auf einen Wert einstellt, der einen vorgegebenen Signalpegel ergibt. Das verstärkte Signal gelangt dann zu der Signalvorverarbeitungsstufe 7, welche die durch die Abschattungen erzeugten Signale formt und dann einem Impulshöhenanalysator des Auswerte- und Anzeigemoduls zuführt. Mit Hilfe dieses Impulshöhenanalysators können die Partikel fest eingestellten Partikelklassen zugeordnet werden, da das Ausmaß der Abschattung von der Partikelgröße abhängt. Nach der Signalbewertung durch den Impulshöhenanalysator gelangen die Signale zu nachgeschalteten Zählern des Auswerte- und Anzeigemoduls 8. Letzterer ist auf eine feste Torzeit oder Zählzeit eingestellt und bringt die in dieser Zeit ermittelte Partikelzahl in den Anzeigefenstern 9 zur Anzeige.

Über die Schnittstelle 11 ist der Partikelzähler von einer Automatisierungseinheit, beispielsweise einer SPS oder einem Steuerrechner, ansprechbar. Außerdem können über die Schnittstelle 11 Daten an eine übergeordnete Leitebene weitergegeben werden. Ferner ist eine Parametrierung und Überwachung des Partikelzählers möglich.

Während sich der Partikelzähler 1 in Betrieb befindet, informieren Überwachungssignale vor Ort, welche im Diagnosefenster 10 sichtbar sind, über den Gerätezustand und einzelne Funktionen. Beispielsweise werden die Größe des Stromes der das Licht der Lichtschranke erzeugenden Diode, das Ausmaß der Signalverstärkung durch die Signalvorverstärkungsstufe 5 und eine erforderlich werdende Reinigung des Meßkanales angezeigt.

Alle in der vorstehenden Beschreibung erwähnten sowie auch die nur allein aus der Zeichnung entnehmbaren Merkmale sind als weitere Ausgestaltungen Bestandteile der Erfindung, auch wenn sie nicht besonders hervorgehoben und insbesondere nicht in den Ansprüchen erwähnt sind.

## Patentansprüche

1. Nach dem Abdunklungsverfahren arbeitender Partikelzähler, insbesondere zum Zählen von opaken Partikeln in einem Fluidstrom eines Hydraulik- oder Schmiersystems, mit einer Lichtschranke, deren Lichtstrahl einen Meßkanal für das Fluid quer zur Kanallängsrichtung durchdringt, und einer dem Empfänger der Lichtschranke nachgeschalteten Auswerteelektronik, dadurch gekennzeichnet, daß
a) die Lichtaustrittsfläche der Lichtschranke durch die Endfläche einer Beleuchtungsfaser (17) und die Lichteintrittsfläche durch die Endfläche einer Empfängerfaser (19) gebildet ist,
b) die Endfläche der Beleuchtungsfaser (17) in einer ersten Begrenzungsfläche des Meßkanals (12) und die konzentrisch zu dieser Endfläche angeordnete Endfläche der Empfängerfaser (19) in einer zur ersten Begrenzungsfläche parallelen zweiten Begrenzungsfläche des Meßkanales (12) liegt,
c) die Beleuchtungsfaser (17) und die Empfängerfaser (19) relativ zueinander und zum Meßkanal (12) unbeweglich und feuchtigkeitsgeschützt in einen Sensorkörper (20) eingebettet sind, der auch den Empfänger der Lichtschranke enthält,
d) die Auswerteelektronik (8) in einem Gehäuse (4) angeordnet ist, das baulich mit dem Sensorkörper (20) vereinigt ist.

2. Zähler nach Anspruch 1, dadurch gekennzeichnet, daß der Meßkanal (12) im Bereich der Lichtschranke einen trapezförmigen Querschnitt hat, wobei die kürzere der beiden zueinander parallelen Seiten eine Länge hat, die gleich dem Durchmesser der Beleuchtungsfaser (17) ist , und die beiden sich an diese kurze Seite anschließenden Seiten (18) mit der Längsachse der Beleuchtungsfaser (17) je einen Winkel (ϑ_{A}) einschließen, der gleich dem Aperturwinkel der Beleuchtungsfaser (17) ist.

3. Zähler nach Anspruch 2, dadurch gekennzeichnet, daß der Durchmesser der Beleuchtungsfaser (17) im Bereich von 200 µ - 300 µ liegt.

4. Zähler nach einem der Ansprüche 1 bis 3, dadurch gekennzeichnet, daß der Abstand der zweiten von der ersten Begrenzungsfläche im Größenbereich des Durchmessers der Beleuchtungsfaser (17) liegt.

5. Zähler nach einem der Ansprüche 1 bis 5, dadurch gekennzeichnet, daß der Durchmesser der Empfängerfaser (19) kleiner, vorzugsweise etwa halb so groß, ist wie der Durchmesser der Beleuchtungsfaser (17).

6. Zähler nach einem der Ansprüche 1 bis 5, dadurch gekennzeichnet, daß als Lichtquelle, deren Licht von der Beleuchtungsfaser (17) zum Meßkanal (12) geleitet wird, eine Infrarotlicht abgebende Leuchtdiode vorgesehen ist.

7. Zähler nach Anspruch 6, dadurch gekennzeichnet, daß die Leuchtdiode in den Sensorkörper (20) integriert ist.

8. Zähler nach einem der Ansprüche 1 bis 7, dadurch gekennzeichnet, daß dem Empfänger ein Signalvorverstärker (5) und diesem eine Signalvorverarbeitungsstufe (7) nachgeschaltet sind.

9. Zähler nach einem der Ansprüche 1 bis 8, dadurch gekennzeichnet, daß die Auswerteelektronik als Auswerte- und Anzeigemodul (8) ausgebildet ist.

10. Zähler nach einem der Ansprüche 1 bis 9, dadurch gekennzeichnet, daß das Gehäuse (4) mit Durchbrüchen versehen ist, die durch ein Diagnosefenster (10) und wenigstens ein Anzeigefenster (9) verschlossen sind.

11. Zähler nach einem der Ansprüche 1 bis 10, dadurch gekennzeichnet, daß der Sensor (3) und das Gehäuse (4) auf einer gemeinsamen Grundplatte (2) angeordnet sind.

12. Zähler nach einem der Ansprüche 1 bis 11, dadurch gekennzeichnet, daß der Ausgang des Meßkanals (12) an einen Volumenstromregler (14) angeschlossen ist.

13. Zähler nach Anspruch 12, dadurch gekennzeichnet, daß zwischen den Sensor (3) und den Volumenstromregler (14) ein Filter (13) geschaltet ist.

14. Zähler nach einem der Ansprüche 1 bis 13, dadurch gekennzeichnet, daß der Sensor (3) an eine baulich mit ihm und dem Gehäuse (4) vereinigte Druckabsenkungseinheit (16) angeschlossen ist.

15. Zähler nach Anspruch 14, dadurch gekennzeichnet, daß die Druckabsenkungseinheit (16) ein Drosselventil und ein Druckventil aufweist.

## Claims

1. Particle counter operating according to the darkening method, in particular for counting opaque particles in a fluid stream of a hydraulic or lubricating system, with a light barrier, whereof the light ray penetrates a measuring channel for the fluid at right angles to the longitudinal direction of the channel, and evaluation electronics connected on the output side of the receiver of the light barrier, characterised in that
a) the light outlet surface of the light barrier is formed by the end surface of an illumination fibre (17) and the light inlet surface is formed by the end surface of a receiver fibre (19),
b) the end surface of the illumination fibre (17) is located in a first boundary surface of the measuring channel (12) and the end surface of the receiver fibre (19) arranged concentrically with respect to this end surface lies in a second boundary surface of the measuring channel (12) parallel to the first boundary surface,
c) the illumination fibre (17) and the receiver fibre (19) are not able to move relative to each other and to the measuring channel (12) and are embedded in a moisture-proof manner in a sensor body (20), which also contains the receiver of the light barrier,
d) the evaluation electronics (8) are located in a housing (4) which is structurally combined with the sensor body (20).

2. Counter according to Claim 1, characterised in that in the region of the light barrier, the measuring channel (12) has a trapezoidal cross-section, the shorter of the two parallel sides having a length which is equal to the diameter of the illumination fibre (17), and the two sides (18) adjoining this short side enclosing with the longitudinal axis of the illumination fibre (17) respectively an angle (ϑ_{A}), which is equal to the aperture angle of the illumination fibre (17).

3. Counter according to Claim 2, characterised in that the diameter of the illumination fibre (17) is in the range of 200µ - 300µ.

4. Counter according to one of Claims 1 to 3, characterised in that the spacing of the second from the first boundary surface lies in the range of size of the diameter of the illumination fibre (17).

5. Counter according to one of Claims 1 to 5, characterised in that the diameter of the receiver fibre (19) is smaller, preferably approximately half as large as the diameter of the illumination fibre (17).

6. Counter according to one of Claims 1 to 5, characterised in that a luminous diode emitting infrared light is provided as the light source, whereof the light is guided by the illumination fibre (17) to the measuring channel (12).

7. Counter according to Claim 6, characterised in that the luminous diode is integrated in the sensor body (20).

8. Counter according to one of Claims 1 to 7, characterised in that connected on the output side of the receiver is a signal pre-amplifier (5) and on the output side thereof a signal pre-processing stage (7).

9. Counter according to one of Claims 1 to 8, characterised in that the evaluation electronics are constructed as an evaluation and indication module (8).

10. Counter according to one of Claims 1 to 9, characterised in that the housing (4) is provided with openings, which are closed by a diagnosis window (10) and at least one indicator window (9).

11. Counter according to one of Claims 1 to 10, characterised in that the sensor (3) and the housing (4) are disposed on a common base plate (2).

12. Counter according to one of Claims 1 to 11, characterised in that the outlet of the measuring channel (12) is connected to a volumetric flow regulator (14).

13. Counter according to Claim 12, characterised in that a filter (13) is connected between the sensor (3) and the volumetric flow regulator (14).

14. Counter according to one of Claims 1 to 13, characterised in that the sensor (3) is connected to a pressure-lowering unit (16) structurally combined with the sensor and the housing (4).

15. Counter according to Claim 14, characterised in that the pressure-lowering unit (16) comprises a restrictor valve and a pressure valve.

## Revendications

1. Compteur de particules, fonctionnant selon le procédé d'opacification, pour le comptage de particules opaques dans le fluide circulant dans un circuit hydraulique ou de lubrification, comportant une cellule photo-électrique dont le rayon lumineux traverse un canal de mesure du fluide transversalement à la direction longitudinale de ce canal, et un dispositif d'analyse électronique monté en aval du récepteur de la cellule photo-électrique, caractérisé en ce que
a) la surface de sortie de la lumière de la cellule photo-électrique est formée par la surface finale d'une fibre d'éclairage (17) et la face d'entrée de la lumière est formée par la face finale d'une fibre de réception (19),
b) la face finale de la fibre d'éclairage (17) se situe dans une première surface de limitation du canal de mesure (12) et la face finale de la fibre de réception (19), concentrique à la surface finale précédente, se situe dans une deuxième surface de délimitation du canal de mesure (12) parallèle à sa première face de délimitation,
c) la fibre d'éclairage (17) et la fibre de réception (19) sont noyées dans un corps détecteur (20) où elles sont protégées contre l'humidité et immobiles l'une par rapport à l'autre ainsi que par rapport au canal de mesure (12), ce corps détecteur (20) contenant également le récepteur de la cellule photo-électrique,
d) le dispositif d'analyse électronique (8) est disposé dans un carter (4), lequel est structurellement réuni au corps détecteur (20).

2. Compteur selon la revendication 1, caractérisé en ce que le canal de mesure (12) présente, au voisinage de la cellule photo-électrique, une section transversale de forme trapèzoïdale, les deux côtés les plus courts parallèles l'un à l'autre de ce trapèze présentant une longueur qui est égale au diamètre de la fibre d'éclairage (10), et les deux côtés (18), faisant suite à ces deux côtés courts renfermant chacun un ongle (ϑ_{A}) avec l'axe longitudinal de la fibre d'éclairage (17), cet angle étant égal à l'angle d'ouverture de la fibre d'éclairage (17).

3. Compteur selon la revendication 2, caractérisé en ce que le diamètre de la fibre d'éclairage (17) se situe dans une plage comprise entre 200 µ et 300 µ.

4. Compteur selon l'une des revendications 1 à 3, caractérisé en ce que la distance entre la deuxième et la première surfaces de délimitation se situe dans un ordre de grandeur du diamètre de la fibre d'éclairage (17).

5. Compteur selon l'une des revendications 1 à 4, caractérisé en ce que le diamètre de la fibre de réception (19) est inférieur, de préférence de moitié, à celui de la fibre d'éclairage (17).

6. Compteur selon l'une des revendications 1 à 5, caractérisé en ce que, pour servir de source lumineuse dont la lumière est conduite par la fibre d'éclairage (17) à travers le canal de mesure (12), est prévue une diode lumineuse émettant une lumière infrarouge.

7. Compteur selon la revendication 6, caractérisé en ce que la diode lumineuse est intégrée dans le corps détecteur (20).

8. Compteur selon l'une des revendications 1 à 7, caractérisé en ce que, en aval du récepteur, est monté un pré-amplificateur de signal (5) et en aval de ce dernier un étage de pré-élaboration du signal (7).

9. Compteur selon l'une des revendications 1 à 8, caractérisé en ce que le dispositif d'analyse électronique est réalisé sous la forme d'un module d'analyse et d'affichage (8).

10. Compteur selon l'une des revendications 1 à 9, caractérisé en ce que le carter (4) est muni de perforations, lesquelles sont fermées par une fenêtre de diagnostic (10) et au moins une fenêtre d'affichage (9).

11. Compteur selon l'une des revendications 1 à 10, caractérisé en ce que le détecteur (3) et le carter (4) sont disposés sur une plaque de base commune (2).

12. Compteur selon l'une des revendications 1 à 11, caractérisé en ce que la sortie du canal de mesure (12) est raccordée à un régulateur de débit (14).

13. Compteur selon la revendication 12, caractérisé en ce que, entre le détecteur (3) et le régulateur de débit (14), est intercalé un filtre (13).

14. Compteur selon l'une des revendications 1 à 13, caractérisé en ce que le détecteur (3) est raccordé à une unité de décompression (16) structurellement réunie à ce détecteur et au carter (4).

15. Compteur selon la revendication 14, caractérisé en ce que l'unité de décompression (16) comporte une soupape de modération et un détendeur.
